# EUROPEAN PATENT SPECIFICATION

(11) **EP 4 464 270 B1**
(45) Date of publication and mention of the grant of the patent: **20.05.2026**
(21) Application number: 24175869.7
(22) Date of filing: 15.05.2024
(51) Int. Cl.: A61B 18/14, A61B 18/00

(54) **ELECTRODES AND ELECTRODE CONFIGURATIONS FOR A BASKET CATHETER**
ELEKTRODEN UND ELEKTRODENKONFIGURATIONEN FÜR EINEN KORBKATHETER
ELECTRODES ET CONFIGURATIONS D'ELECTRODES POUR UN CATHETER A PANIER

(30) Priority: 16.05.2023 US 202363502543 P; 18.04.2024 US 202418639061
(43) Date of publication of application: 20.11.2024
(73) Proprietor: Biosense Webster (Israel) Ltd., 2066717 Yokneam (IL)
(72) Inventor: XU, Guo, Irvine, 92618 (US); RORICK, Kevin, Irvine, 92618 (US); JIMENEZ, Jose, Irvine, 92618 (US)
(74) Representative: Carpmaels & Ransford LLP

(56) References cited:
- EP-A1- 4 215 144
- CN-A- 114 027 967
- US-A1- 2022 071 694

## Description

### FIELD

The present invention relates generally to medical devices, and in particular medical probes with electrodes, and further relates to, but not exclusively, medical probes suitable for mapping and ablation of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Regions of cardiac tissue can be mapped by a medical probe to identify the abnormal electrical signals. The same or different medical probe can be used to perform ablation. Some example probes include a number of spines with electrodes positioned thereon. The electrodes are generally attached to the spines and the spines are configured to bow radially outward when deployed from a sheath. The electrodes are then brought into contact with tissue for mapping or ablation of the tissue. The electrodes must generally be arranged on the spines in such a way that electrodes on adjacent spines are offset from each other. This is due to the limited space within the sheath and because the electrodes cannot fit together within the sheath if electrodes on adjacent spines are aligned with each other. Offset alignment of electrodes, however, results in fewer electrodes being attached to the spines which can make mapping and ablation less accurate. Accordingly, there is a need in the art for electrode designs that will enable electrodes to be aligned on adjacent spines and permit for a greater number of electrodes to be attached to the spines while still being able to fit within a sheath. These and other problems can be addressed by the technology disclosed herein.

CN-A-114 027 967 discloses an electrode for a medical probe comprising a conductive surface forming a generally triangular shape.

### SUMMARY

There is provided, in accordance with the disclosed technology, an electrode comprising a body including a first end and a second end. The body can extend along a longitudinal axis from the first end to the second end. The body can be configured for attachment to a spine of an expandable basket assembly such that the first end is oriented for contacting tissue when the expandable basket assembly is in an expanded configuration. The first end can comprise a conductive surface forming a generally triangular shape and an area that is greater than the second end. The body can converge from the first end to the second end along the longitudinal axis.

A medical probe comprising a tubular shaft including a proximal end and a distal end, the tubular shaft extending along a longitudinal axis, and an expandable basket assembly coupled to the distal end of the tubular shaft. The expandable basket assembly comprising a plurality of spines configured to bow radially outward from the longitudinal axis when the expandable basket assembly is in an expanded configuration and a plurality of electrodes attached to the plurality of spines Each electrode of the plurality of electrodes comprising a body including a first end and a second end, the body being attached to a respective spine of the plurality of spines such that the first end is oriented for contacting tissue when the expandable basket assembly is in the expanded configuration. The first end can comprise a conductive surface forming a generally triangular shape and an area that is greater than the second end and the body can converge from the first end to the second end.

The disclosed technology can include a medical probe comprising a tubular shaft including a proximal end and a distal end. The tubular shaft can extend along a longitudinal axis. The medical probe can further comprise an expandable basket assembly coupled to the distal end of the tubular shaft. The expandable basket assembly can comprise a plurality of spines configured to bow radially outward from the longitudinal axis when the expandable basket assembly is in an expanded configuration. The expandable basket can comprise a plurality of electrodes attached to the plurality of spines.

Each electrode of the plurality of electrodes can include a body comprising a tetrahedral shape. The tetrahedral shape can comprise an outwardly-facing end and an inwardly-facing end. The outwardly-facing end can comprise a conductive surface including an area that is greater than an area of the inwardly-facing end and be configured to contact tissue when the expandable basket assembly is in the expanded configuration. The body can converge from the outwardly-facing end to the inwardly-facing end.

### BRIEF DESCRIPTION OF THE DRAWINGS

The present disclosure will be more fully understood from the following detailed description of embodiments thereof, taken together with the drawings, in which:
FIG. 1 is a schematic pictorial illustration of a medical system including a medical probe with a distal end comprising electrodes, in accordance with the disclosed technology;
FIG. 2 is a schematic pictorial illustration of a basket catheter comprising electrodes, in accordance with the disclosed technology;
FIG. 3A is a perspective view of an electrode, in accordance with the disclosed technology;
FIG. 3B is a front view of the electrode, in accordance with the disclosed technology;
FIG. 3C is a section view of the electrode of FIG. 3B taken along line A-A, in accordance with the disclosed technology;
FIG. 3D is a top view of the electrode, in accordance with the disclosed technology;
FIG. 3E is a section view of the electrode of FIG. 3D take along line B-B, in accordance with the disclosed technology;
FIG. 4A is a schematic pictorial illustration of the medical probe, in accordance with the disclosed technology;
FIG. 4B is a schematic pictorial illustration of the medical probe in a sheath, in accordance with the disclosed technology;
FIG. 5 is a perspective view of a plurality of spines and electrodes of the medical probe, in accordance with the disclosed technology;
FIG. 6A is a sideview of a grouping of electrodes, in accordance with the disclosed technology;
FIG. 6B is a top view of a grouping of electrodes, in accordance with the disclosed technology;
FIG. 6C is a top view of another grouping of electrodes, in accordance with another example of the disclosed technology; and
FIG. 7 is a section view of the spines and electrodes taken along line C-C of FIG. 6B, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, method or uses and devices that can be used for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1.

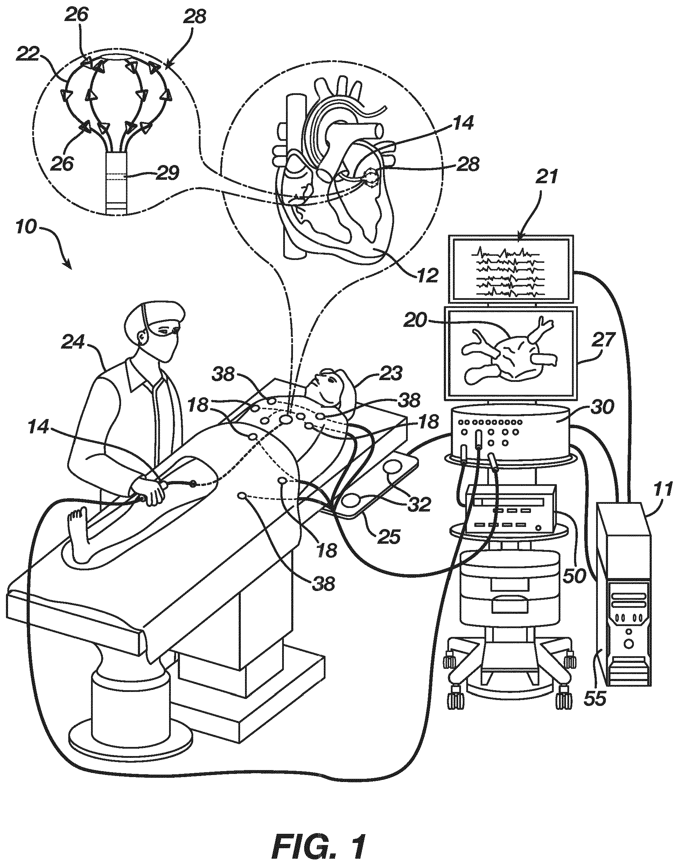

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing and ablating is illustrated herein. Physician 24 brings a distal tip 28 (sometimes referred to herein as basket catheter 28 or expandable basket assembly) of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over a plurality of spines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

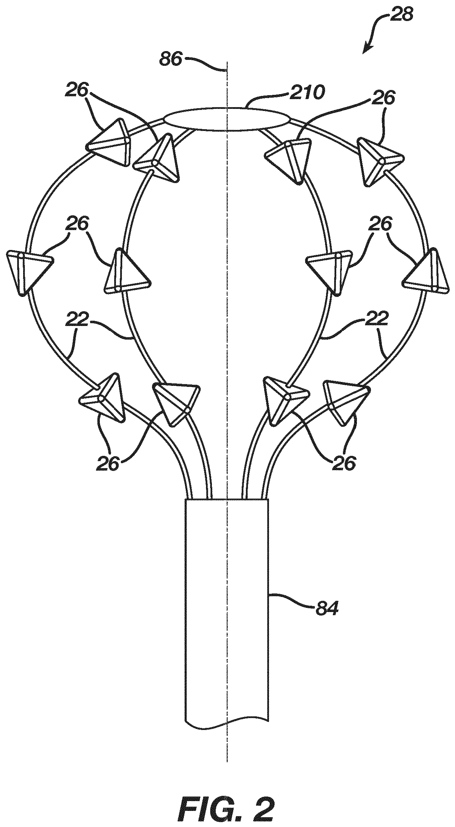

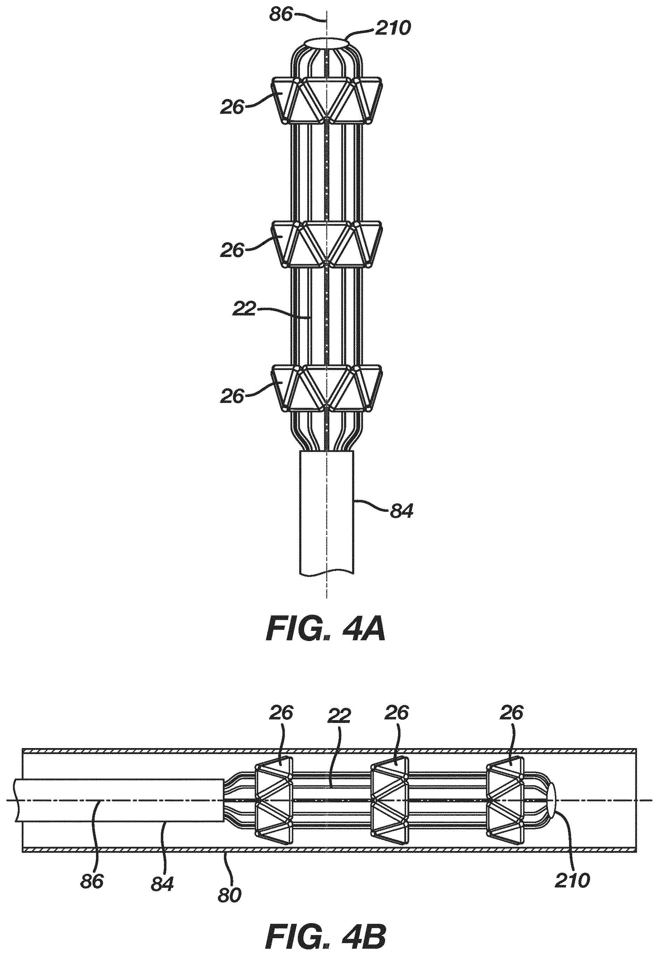

FIG. 2 is a schematic pictorial illustration showing a perspective view of a basket catheter 28 with electrodes 26 in an expanded form, such as by being advanced out of a sheath 80 (shown in FIG. 4B), in accordance the disclosed technology. As shown in FIG. 2, the basket catheter 28 can include a plurality of spines 22 that can bow radially outward from a longitudinal axis 86 when in an expanded form. The spines 22 can be formed from a biocompatible resilient material such as nitinol such that the spines 22 are naturally biased to expand outwardly to the expanded form.

Each spine 22 can include one or more electrodes 26 attached thereto. As will be described in greater detail herein, the electrodes 26 can be configured such that the electrodes 26 can be disposed on the spines 22 in alignment on adjacent spines 22 as shown in FIG. 2. Thus, the basket catheter 28 of the disclosed technology can be configured to include a greater number of electrodes 26 than previous catheter designs. The electrodes 26 can be configured such that the electrodes can collapse together in a tight configuration as shown in FIGs. 4A-7. The electrodes 26 described herein can be configured for mapping and/or ablation of tissue.

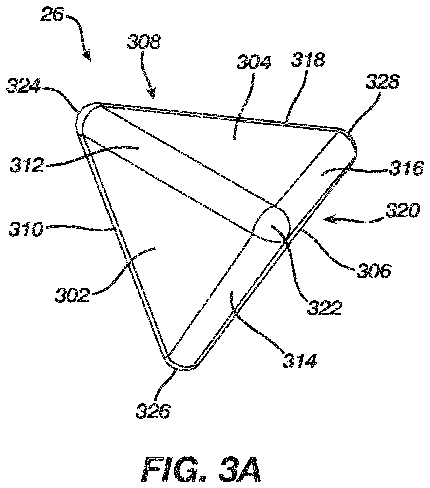

FIG. 3A is a perspective view of an electrode 26, in accordance with the disclosed technology. As shown, the electrodes 26 can each have a generally tetrahedral shape. In this way, the electrodes 26 can be attached to the spines 22 in such a way that an outwardly-facing surface 302 (also referred to herein as a "first end" and the "first face") of the electrode 26 can have a greater surface area than an inwardly-facing end 328 of the electrode 26 (also referred to herein as the "second end" or the "fourth corner"). Because of the generally tetrahedral shape, the electrodes 26, when attached to the spines 22 and arranged on the basket catheter 28, can be configured to collapse together in a tight configuration like wedges of an orange. Thus, the electrodes 26 enable the basket catheter 28 to comprise a greater number of electrodes 26 while still being able to collapse and fit within a sheath 80.

All or some of the electrode 26 can comprise a conductive material. For example, all of the electrode 26 can be made entirely from gold, silver, platinum, palladium, stainless steel (and their respective alloys). Alternatively, only a portion of the electrode 26 (e.g., the first face 302) can be made from the just-recited materials. These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12. The electrodes can be configured to deliver electrical pulses having a peak voltage of at least 900 volts (V).

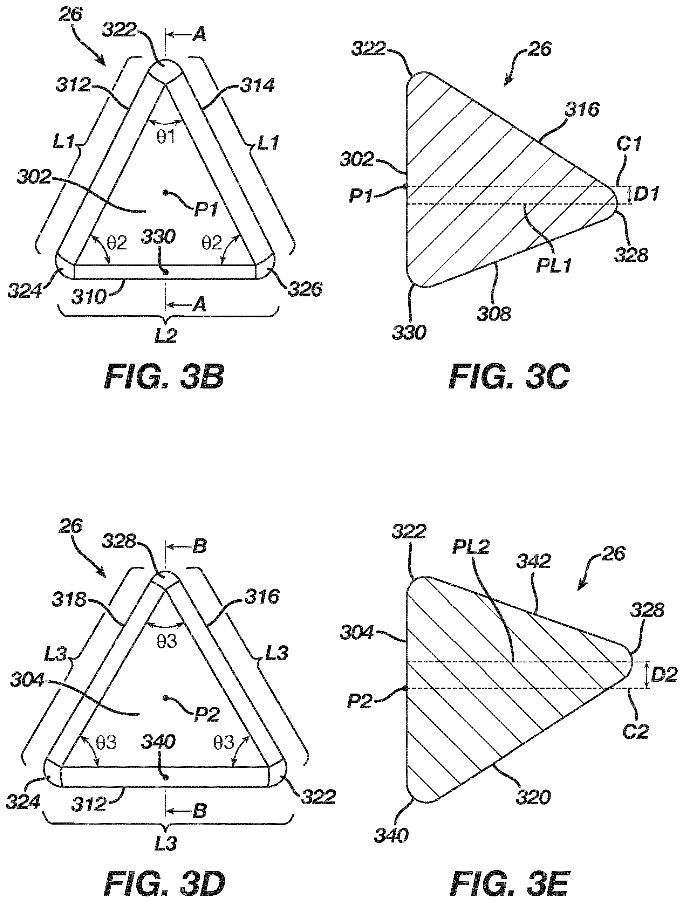

The outwardly-facing surface 302 can be configured for contacting tissue and, thus, by having a greater surface area than the inwardly-facing end 328, the outwardly-facing surface 302 can be better configured for detecting electrophysiological signals and/or for providing ablative energy to the tissue. Furthermore, the electrodes 26 can have a plurality of faces forming the generally tetrahedral shape. For example, the electrodes 26 can have at least a first face 302 (the outwardly-facing side), a second face 304, a third face 306, and a fourth face 308 (not visible in the view shown in FIG. 3A). Furthermore, the electrodes 26 can have a plurality of edges between the plurality of faces. For example, the electrodes 26 can have a first edge 310, a second edge 312, a third edge 314, a fourth edge 316, a fifth edge 318, and a sixth edge 320 (not fully visible in FIG. 3A). Each of the first edge 310, a second edge 312, a third edge 314, a fourth edge 316, a fifth edge 318, and a sixth edge 320 can be rounded to prevent damage to tissue and other components of the medical probe (e.g., the sheath 80). The plurality of edges can converge together at a plurality of corners. The plurality of corners can include a first corner 322, a second corner 324, a third corner 326, and a fourth corner 328 (also referred to herein as "second end" or "inwardly-facing end"). Each of the faces, edges, and corners just described and shown in FIG. 3A correspond to like faces, edges, and corners shown in FIGs. 3B-3E.

Turning now to FIGs. 3B-3E, FIG. 3B is a front view of the electrode 26, FIG. 3C is a section view of the electrode of FIG. 3B, FIG. 3D is a top view of the electrode, and FIG. 3E is a section view of the electrode of FIG. 3D, in accordance with the disclosed technology. The views shown in FIGs. 3B-3E illustrate the various features of the electrodes 26, including the general angles and proportions of an example electrodes. One of skill in the art will appreciate that the views of the electrode shown and described in relation to FIGs. 3B-3E are offered for illustrative purposes and should not be construed as limiting. Other electrodes 26 having varying shapes, angles, proportions, etc. can similarly be construed within the scope of this disclosure.

As shown in FIG. 3B, the electrode 26 can have a first face 302 (outwardly-facing side) that is configured to contact tissue. The first face 302 can be formed generally in the shape of a triangle. In this non-limiting example, the first face 302 is shown as forming an isosceles triangle with the second edge 312 and the third edge 314 having a first length L1 that is different than the length L2 of the first edge 310. Because the first face 302 forms an isosceles triangle, the first angle θ1 between the second edge 312 and the third edge 314 can be different than the second angle θ2 between the first edge 310 and the second edge 312 which is equal to the angle θ2 between the first edge 310 and the third edge 312. As will be appreciated, however, the various angles and lengths of the faces can be configured according to various other examples. For instance, the first face 302 can form an acute triangle, an obtuse triangle, an equilateral triangle, scalene triangle, right triangle, etc. Furthermore, although the electrode 26 is shown as having a first face 302 (and other faces, 304, 306, 308) forming a triangle, it will be appreciated that the one or more faces of the triangle can form shapes other than triangles. Thus, various other permutations of the disclosed technology can be configured such that the outwardly-facing side has a greater surface area than the inwardly-facing side such that the electrodes 26 can collapse together to form a tight grouping of electrodes 26 capable of fitting within the sheath 80.

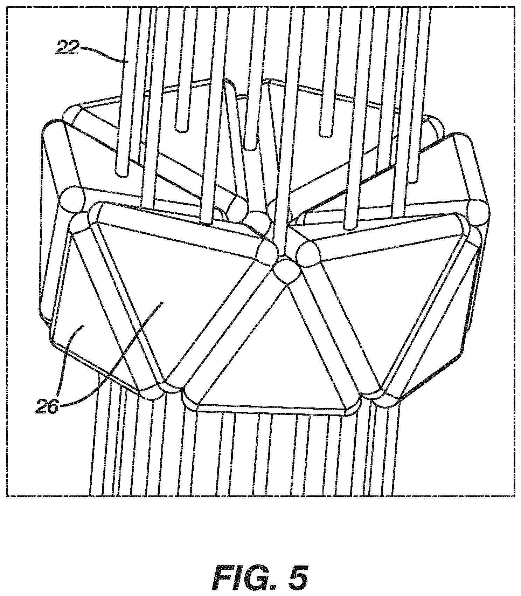

FIG. 3C is a cross-sectional view of the electrode 26 taken along line A-A of FIG. 3B. Line A-A extends from the fourth corner 328 (inwardly-facing end) through the middle of the first face 302 to a point 330 disposed in the middle of the first edge 310. As shown in FIG. 3C, the electrode 26 can be configured such that the fourth corner 328 (inwardly-facing end) can be offset a distance D1 from a centerline C1 extending perpendicularly from a point P1 that is at the center of the triangle formed by the first face 302. That is, the center line C1 extending perpendicularly to the first face 302 from a first point P1 at a center of the first face 302 is spaced a distance D1 apart from a line PL1 parallel to the first line and extending through a second point at a center of the fourth corner 328 ("inwardly-facing end" or "second end"). Stated otherwise, the electrode 26 can form a tetrahedron that is not a regular tetrahedron. In this way, the fourth corner 328 of the electrodes 26 can be configured to better fit together when the electrodes 26 are grouped together as will be described in greater detail in relation to FIGs. 5-7.

As shown in FIG. 3D, the second face 304 of the electrode 26 can form an equilateral triangle. That is, the second edge 312, the fourth edge 316, and the fifth edge 318 can each have the same length L3 and form third angles θ3 between each of the second edge 312, the fourth edge 316, and the fifth edge 318 that are all equal to each other. As before, the configuration of the second face 304 shown in FIG. 3D is offered for illustrative purposes and the second face 304 can be configured according to various other permutations.

FIG. 3E is a cross-sectional view of the electrode 26 taken along line B-B of FIG. 3D. Line B-B extends from the fourth corner 328 (inwardly-facing end) through the middle of the second face 304 to a point 340 disposed in the middle of the second edge 312. Similar to the cross-section of the electrode 26 shown in FIG. 3C, the cross-sectional view of FIG. 3E illustrates that the second end 328 can be disposed such that it is offset in alignment a distance D2 from a centerline C2 extending perpendicularly from a point P2 at the center of the second face 304. That is, a centerline C2 extending perpendicularly to the second face 304 from a second point P2 at a center of the second face 304 is spaced a distance D2 apart from a line PL2 parallel to the center line C2 and extending through a point at a center of the fourth corner 328 ("inwardly-facing end" or "second end"). In this way, the fourth corner 328 (inwardly-facing end) of the electrodes 26 can be configured to better fit together when the electrodes 26 are grouped together as will be described in greater detail in relation to FIGs. 5-7. As will be appreciated, the distances D1 and D2 can be changed to affect the configuration of the tetrahedral shape of the electrode 26 as suitable for the particular application.

As shown in FIG. 4A, the basket catheter 28 can include a plurality of flexible spines 22 that are formed at the end of a tubular shaft 84 and are connected at a proximal end to the tubular shaft 84 and at a distal end to a distal hub 210. During a medical procedure, physician 34 can deploy basket catheter 28 by extending tubular shaft 84 from sheath 80 causing the basket catheter 28 to exit the sheath 80 and transition to the expanded form (as shown in FIG. 2). As shown in FIGs. 4A and 4B, because the electrodes 26 are configured according to the shapes shown and described herein, the electrodes 26 can be configured to collapse together and form a tight group for delivery from, and retraction into, the sheath.

Spines 22 may have elliptical (e.g., circular) or rectangular (that may appear to be flat) cross-sections, and include a flexible, resilient material (e.g., a shape-memory alloy such as nickel-titanium, also known as Nitinol or other materials such as cobalt chromium, stainless steel, titanium, or even a polymer material) forming a strut as will be described in greater detail herein. The disclosed technology can be applicable to basket catheter 28 formed from a single spine 22 or multiple spines 22 with each spine 22 being attached at both ends (as just described).

The spines 22 can be formed from a single sheet of planar material. In some examples, the spines 22 can be formed from the single sheet of planar material such that the spines 22 converge toward a central intersection. In other examples, the spines 22 can be formed individually and then attached at both ends as shown and just described.

As will be appreciated, the spine 22 can be electrically isolated from the electrode 26 to prevent arcing from the electrode 26 to the spine 22. For example, insulative jackets (not shown) can be positioned between the spine 22 and the electrode 26, but one of skill in the art will appreciate that other insulative coverings are contemplated. For example, an insulative coating can be applied to the spine 22, the electrodes 26, or both. The insulative jackets can be made from a biocompatible, electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets may also include one or more additives or fillers, such as, for example, polytetrafluoroethylene (PTFE), boron nitride, silicon nitride, silicon carbide, aluminum oxide, aluminum nitride, zinc oxide, and the like.

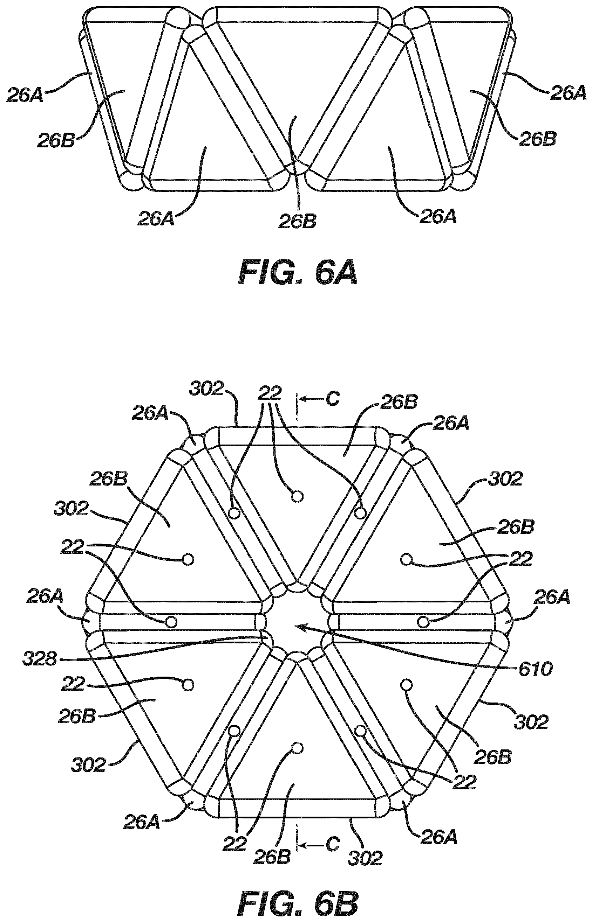

FIG. 5 illustrates a perspective view of the electrodes 26 attached to the spines 22 in a collapsed form. As shown, the electrodes 26 can be attached to the spines 22 such that a majority of the electrode 26 is disposed on an outer-facing side of the spine 22. As better shown in FIGs. 6A and 6B, the electrodes 26 can be arranged in an alternating first orientation 26A and second orientation 26B. The second orientation 26B can be flipped 180 degrees from first orientation 26A such that the electrodes 26 are better able to fit together when in the collapsed form (as shown in FIGs. 4A-7). Furthermore, as can be seen in FIG. 6B, the first face 302 can be arranged in an outwardly-facing orientation while the fourth corner 328 (second end) is arranged in an inwardly-facing orientation. In the example shown in FIG. 6B, the fourth corners 328 can converge and define a lumen 610 extending through the grouping of electrodes 26. The lumen 610, for example, can be useful for ensure sufficient room remains for the electrodes 26 to align together with some variance in alignment. Furthermore, in some examples, the lumen 610 can be configured to permit irrigation fluid to flow therethrough if, for example, it is desirable for irrigation fluid to flow through the probe even when the basket catheter 28 is withdrawn into the sheath 80.

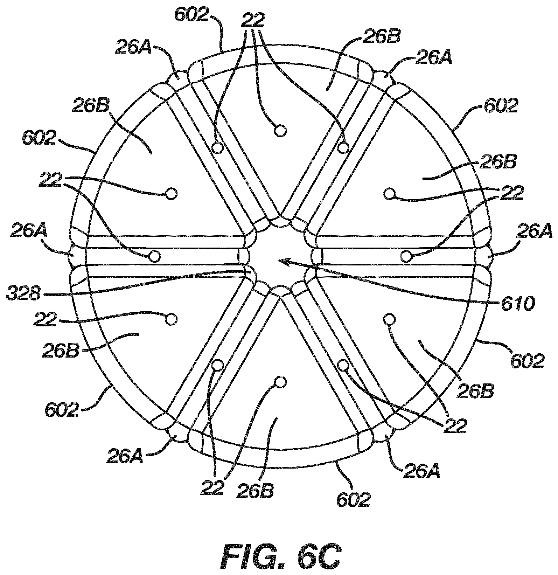

FIG. 6C illustrates another example arrangement of electrodes 26 in the collapsed configuration. In this example, the electrodes 26 comprise a rounded first face 602 that can help to prevent the electrodes 26 from damaging tissue or other components of the medical probe (e.g., the interior of the sheath, the hemostasis valve, etc.). That is, the rounded first face 602 can comprise a compound curved surface that presents a rounded profile with respect to the longitudinal axis 86. As shown, the rounded first face 602 can be outwardly-facing and configured to contact tissue. Furthermore, the rounded first face 602 can make it easier to withdraw the basket catheter 28 into the sheath 80.

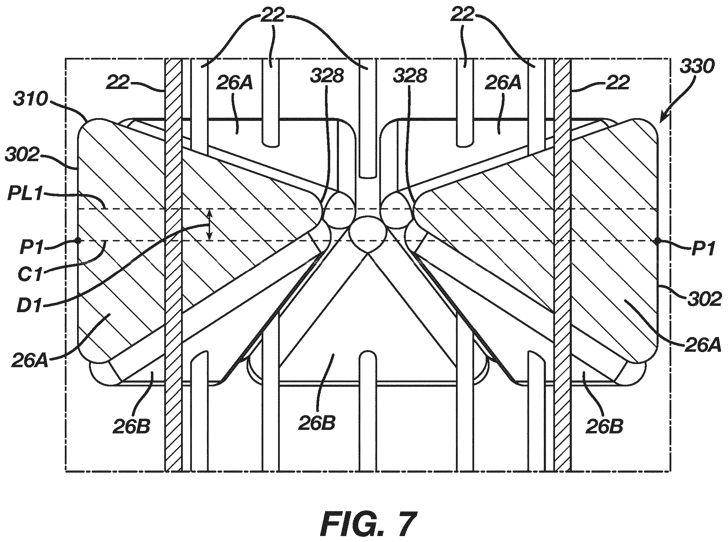

FIG. 7 is a section view of the spines 22 and electrodes 26 taken along line C-C of FIG. 6B. As shown in FIG. 7, and previously described in relation to FIG. 3C, the electrode 26 can be configured such that the fourth corner 328 (inwardly-facing end) can be offset a distance D1 from a centerline C1 extending perpendicularly from a point P1 that is at the center of the triangle formed by the first face 302. Thus, the second end 328 of each electrode 26 can converge toward a point in the middle of the electrode 26 grouping that is offset from a center of the first face 302. Furthermore, as shown, the first edge 310 can be oriented generally perpendicular with respect to the spine 22. As will be appreciated, however, the electrodes 26 can be configured in various other configurations that enable the electrodes 26 to collapse together in a compact manner thereby permitting electrodes 26 to be attached to adjacent spines 22 while still being able to fit compactly within the sheath 80.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An electrode (26) for a medical probe, the electrode comprising:
a body including a first end (302) and a second end (328), the body extending along a longitudinal axis from the first end to the second end, the body configured for attachment to a spine (22) of an expandable basket assembly such that the first end is oriented for contacting tissue when the expandable basket assembly is in an expanded configuration,
the first end comprising a conductive surface forming a generally triangular shape and an area that is greater than the second end, and
the body converging from the first end to the second end along the longitudinal axis.

2. A medical probe comprising:
a tubular shaft (84) including a proximal end and a distal end, the tubular shaft extending along a longitudinal axis; and
an expandable basket assembly (28) coupled to the distal end of the tubular shaft, the expandable basket assembly comprising:
a plurality of spines (22) configured to bow radially outward from the longitudinal axis when the expandable basket assembly is in an expanded configuration; and
a plurality of electrodes (26) attached to the plurality of spines, each electrode being an electrode according to claim 1.

3. The electrode of claim 1 or the medical probe of claim 2, the body comprising a tetrahedron.

4. The electrode or medical probe of claim 3, wherein the second end is misaligned with the first end such that a first line extending perpendicularly to the conductive surface from a first point at a center of the first end is spaced a distance apart from a second line parallel to the first line and extending through a second point at a center of the second end.

5. The electrode of any of claims 1, 3 or 4, the body being configured for attachment to the spine such that a first edge of the generally triangular shape is oriented perpendicular to the spine.

6. The electrode of any of claims 1, 3, 4 or 5, the electrode being configured for ablation of the tissue.

7. The electrode of any of claims 1, 3, 4, 5 or 6, the conductive surface forming the generally triangular shape comprising a compound curved surface that presents a rounded profile with respect to the longitudinal axis.

8. The electrode of any of claims 1, 3, 4, 5, 6 or 7, the electrode being configured for mapping electrophysiological characteristics of the tissue.

9. The medical probe of any of claims 2-4, wherein the plurality of electrodes are attached to each of the spines such that each adjacent spine comprises an electrode, and wherein the plurality of electrodes are aligned in a direction perpendicular to the longitudinal axis.

10. The medical probe of claim 9, the body of each electrode of the plurality of electrodes being attached to the respective spine such that a first edge of the generally triangular shape is oriented perpendicular to the spine, optionally
wherein a first electrode of the plurality of electrodes is attached to the respective spine in a first orientation such that the first edge is at a distal end of the body, and
wherein a second electrode of the plurality of electrodes is attached to an adjacent spine of the plurality of spines in a second orientation such that the first edge is at a proximal end of the body, further optionally
wherein the plurality of electrodes are attached to the plurality of spines such that the plurality of electrodes are attached to adjacent spines in alternating first and second orientations.

11. A medical probe according to claim 2, wherein each body comprises a tetrahedral shape, each first end comprises an outwardly-facing end and each second end comprises an inwardly-facing end.

12. The medical probe of claim 11, wherein the inwardly-facing end is misaligned with the outwardly-facing end such that a first line extending perpendicularly to the conductive surface from a first point at a center of the first end is spaced a distance apart from a second line parallel to the first line and extending through a second point at a center of the second end.

13. The medical probe of claim 11 or claim 12, wherein the plurality of electrodes are attached to each of the spines such that each adjacent spine comprises an electrode, and wherein the plurality of electrodes are aligned in a direction perpendicular to the longitudinal axis.

14. The medical probe of claim 11, the body of each electrode of the plurality of electrodes being attached to the respective spine such that a first edge of the conductive surface is oriented perpendicular to the spine.

15. The medical probe of claim 13, wherein a first electrode of the plurality of electrodes is attached to the respective spine in a first orientation such that a first edge of the conductive surface is at a distal end of the body, and
wherein a second electrode of the plurality of electrodes is attached to an adjacent spine of the plurality of spines in a second orientation such that a first edge of the conductive surface is at a proximal end of the body, optionally
wherein the plurality of electrodes are attached to the plurality of spines such that the plurality of electrodes are attached to adjacent spines in alternating first and second orientations.

## Patentansprüche

1. Elektrode (26) für eine medizinische Sonde, die Elektrode umfassend:
einen Körper, einschließlich eines ersten Endes (302) und eines zweiten Endes (328), wobei sich der Körper entlang einer Längsachse von dem ersten Ende zu dem zweiten Ende erstreckt, wobei der Körper zur Befestigung an einer Strebe (22) einer expandierbaren Korbanordnung derart konfiguriert ist, dass das erste Ende zum Kontakt mit Gewebe ausgerichtet ist, wenn sich die expandierbare Korbanordnung in einer expandierten Konfiguration befindet,
wobei das erste Ende eine leitfähige Oberfläche umfasst, die eine im Allgemeinen dreieckige Form bildet und eine Fläche aufweist, die größer ist als das zweite Ende, und
der Körper sich vom ersten Ende zum zweiten Ende entlang der Längsachse verjüngt.

2. Medizinische Sonde, umfassend:
einen rohrförmigen Schaft (84), einschließlich eines proximalen Endes und eines distalen Endes, wobei sich der rohrförmige Schaft entlang einer Längsachse erstreckt; und
eine expandierbare Korbanordnung (28), die mit dem distalen Ende des rohrförmigen Schafts gekoppelt ist, die expandierbare Korbanordnung umfassend:
eine Vielzahl von Streben (22), die konfiguriert sind, um sich von der Längsachse radial nach außen zu wölben, wenn sich die expandierbare Korbanordnung in einer expandierten Konfiguration befindet; und
eine Vielzahl von Elektroden (26), die an der Vielzahl von Streben befestigt sind, wobei jede Elektrode eine Elektrode nach Anspruch 1 ist.

3. Elektrode nach Anspruch 1 oder medizinische Sonde nach Anspruch 2, wobei der Körper einen Tetraeder umfasst.

4. Elektrode oder medizinische Sonde nach Anspruch 3, wobei das zweite Ende gegenüber dem ersten Ende derart versetzt ist, dass eine erste Linie, die sich von einem ersten Punkt in der Mitte des ersten Endes senkrecht zur leitfähigen Oberfläche erstreckt, von einer zweiten Linie beabstandet ist, die parallel zur ersten Linie verläuft und sich durch einen zweiten Punkt in der Mitte des zweiten Endes erstreckt.

5. Elektrode nach einem der Ansprüche 1, 3 oder 4, wobei der Körper zur Befestigung an der Strebe derart konfiguriert ist, dass eine erste Kante der im Allgemeinen dreieckigen Form senkrecht zur Strebe ausgerichtet ist.

6. Elektrode nach einem der Ansprüche 1, 3, 4 oder 5, wobei die Elektrode zur Ablation des Gewebes konfiguriert ist.

7. Elektrode nach einem der Ansprüche 1, 3, 4, 5 oder 6, wobei die leitfähige Oberfläche, die die im Allgemeinen dreieckige Form bildet, eine zusammengesetzte gekrümmte Oberfläche umfasst, die ein abgerundetes Profil in Bezug auf die Längsachse präsentiert.

8. Elektrode nach einem der Ansprüche 1, 3, 4, 5, 6 oder 7, wobei die Elektrode zur Abbildung elektrophysiologischer Eigenschaften des Gewebes konfiguriert ist.

9. Medizinische Sonde nach einem der Ansprüche 2 bis 4, wobei die Vielzahl von Elektroden an jeder der Streben derart befestigt ist, dass jede benachbarte Strebe eine Elektrode umfasst, und wobei die Vielzahl von Elektroden in einer Richtung senkrecht zur Längsachse ausgerichtet ist.

10. Medizinische Sonde nach Anspruch 9, wobei der Körper jeder Elektrode der Vielzahl von Elektroden an der jeweiligen Strebe derart befestigt ist, dass eine erste Kante der im Allgemeinen dreieckigen Form senkrecht zur Strebe ausgerichtet ist, gegebenenfalls
wobei eine erste Elektrode der Vielzahl von Elektroden an der jeweiligen Strebe in einer ersten Orientierung derart befestigt ist, dass die erste Kante an einem distalen Ende des Körpers liegt, und
wobei eine zweite Elektrode der Vielzahl von Elektroden an einer benachbarten Strebe der Vielzahl von Streben in einer zweiten Orientierung derart befestigt ist, dass die erste Kante an einem proximalen Ende des Körpers liegt, ferner gegebenenfalls
wobei die Vielzahl von Elektroden an der Vielzahl von Streben derart befestigt ist, dass die Vielzahl von Elektroden an benachbarten Streben in alternierenden ersten und zweiten Ausrichtungen befestigt ist.

11. Medizinische Sonde nach Anspruch 2, wobei jeder Körper eine tetraedrische Form umfasst, jedes erste Ende ein nach außen gerichtetes Ende umfasst und jedes zweite Ende ein nach innen gerichtetes Ende umfasst.

12. Medizinische Sonde nach Anspruch 11, wobei das nach innen gerichtete Ende gegenüber dem nach außen gerichteten Ende derart versetzt ist, dass eine erste Linie, die sich von einem ersten Punkt in der Mitte des ersten Endes senkrecht zur leitfähigen Oberfläche erstreckt, von einer zweiten Linie beabstandet ist, die parallel zur ersten Linie verläuft und sich durch einen zweiten Punkt in der Mitte des zweiten Endes erstreckt.

13. Medizinische Sonde nach Anspruch 11 oder Anspruch 12, wobei die Vielzahl von Elektroden an jeder der Streben derart befestigt ist, dass jede benachbarte Strebe eine Elektrode umfasst, und wobei die Vielzahl von Elektroden in einer Richtung senkrecht zur Längsachse ausgerichtet ist.

14. Medizinische Sonde nach Anspruch 11, wobei der Körper jeder Elektrode der Vielzahl von Elektroden an der jeweiligen Strebe derart befestigt ist, dass eine erste Kante der leitfähigen Oberfläche senkrecht zur Strebe ausgerichtet ist.

15. Medizinische Sonde nach Anspruch 13, wobei eine erste Elektrode der Vielzahl von Elektroden an der jeweiligen Strebe in einer ersten Orientierung derart befestigt ist, dass eine erste Kante der leitfähigen Oberfläche an einem distalen Ende des Körpers liegt, und
wobei eine zweite Elektrode der Vielzahl von Elektroden an einer benachbarten Strebe der Vielzahl von Streben in einer zweiten Orientierung derart befestigt ist, dass eine erste Kante der leitfähigen Oberfläche an einem proximalen Ende des Körpers liegt, gegebenenfalls
wobei die Vielzahl von Elektroden an der Vielzahl von Streben derart befestigt ist, dass die Vielzahl von Elektroden an benachbarten Streben in alternierenden ersten und zweiten Ausrichtungen befestigt ist.

## Revendications

1. Électrode (26) destinée à une sonde médicale, l'électrode comprenant :
un corps comportant une première extrémité (302) et une seconde extrémité (328), le corps s'étendant le long d'un axe longitudinal à partir de la première extrémité jusqu'à la seconde extrémité, le corps étant conçu pour fixation à un élément d'armature (22) d'un ensemble panier expansible de telle sorte que la première extrémité est orientée pour un contact avec un tissu lorsque l'ensemble panier expansible est dans une configuration expansée,
la première extrémité comprenant une surface conductrice formant une forme généralement triangulaire et une aire qui est supérieure à la seconde extrémité, et
le corps convergeant de la première extrémité à la seconde extrémité le long de l'axe longitudinal.

2. Sonde médicale comprenant :
une tige tubulaire (84) comportant une extrémité proximale et une extrémité distale, la tige tubulaire s'étendant le long d'un axe longitudinal ; et
un ensemble panier expansible (28) accouplé à l'extrémité distale de la tige tubulaire, l'ensemble panier expansible comprenant :
une pluralité d'éléments d'armature (22) conçus pour s'incurver radialement vers l'extérieur à partir de l'axe longitudinal lorsque l'ensemble panier expansible est dans une configuration expansée ; et
une pluralité d'électrodes (26) fixées à la pluralité d'éléments d'armature, chaque électrode étant une électrode selon la revendication 1.

3. Électrode selon la revendication 1 ou sonde médicale selon la revendication 2, le corps comprenant un tétraèdre.

4. Électrode ou sonde médicale selon la revendication 3, dans laquelle la seconde extrémité n'est pas alignée avec la première extrémité de telle sorte qu'une première ligne s'étendant perpendiculairement à la surface conductrice à partir d'un premier point au niveau d'un centre de la première extrémité est espacée à une certaine distance par rapport à une seconde ligne parallèle à la première ligne et s'étendant à travers un second point au niveau d'un centre de la seconde extrémité.

5. Électrode selon l'une quelconque des revendications 1, 3 ou 4, le corps étant conçu pour fixation à l'élément d'armature de telle sorte qu'un premier bord de la forme généralement triangulaire est orienté perpendiculaire à l'élément d'armature.

6. Électrode selon l'une quelconque des revendications 1, 3, 4 ou 5, l'électrode étant conçue pour une ablation du tissu.

7. Électrode selon l'une quelconque des revendications 1, 3, 4, 5 ou 6, la surface conductrice formant la forme généralement triangulaire comprenant une surface courbe composée qui présente un profil arrondi par rapport à l'axe longitudinal.

8. Électrode selon l'une quelconque des revendications 1, 3, 4, 5, 6 ou 7, l'électrode étant configurée pour une cartographie de caractéristiques électrophysiologiques du tissu.

9. Sonde médicale selon l'une quelconque des revendications 2 à 4, dans laquelle la pluralité d'électrodes sont fixées à chacun des éléments d'armature de telle sorte que chaque élément d'armature adjacent comprend une électrode, et dans laquelle la pluralité d'électrodes sont alignées dans une direction perpendiculaire à l'axe longitudinal.

10. Sonde médicale selon la revendication 9, le corps de chaque électrode de la pluralité d'électrodes étant fixé à l'élément d'armature respectif de telle sorte qu'un premier bord de la forme généralement triangulaire est orienté perpendiculaire à l'élément d'armature, facultativement
dans laquelle une première électrode de la pluralité d'électrodes est fixée à l'élément d'armature respectif dans une première orientation de telle sorte que le premier bord est au niveau d'une extrémité distale du corps, et
dans laquelle une seconde électrode de la pluralité d'électrodes est fixée à un élément d'armature adjacent de la pluralité d'éléments d'armature dans une seconde orientation de telle sorte que le premier bord est au niveau d'une extrémité proximale du corps, facultativement en outre
dans laquelle la pluralité d'électrodes sont fixées à la pluralité d'éléments d'armature de telle sorte que la pluralité d'électrodes sont fixées à des éléments d'armature adjacents dans première et seconde orientations alternées.

11. Sonde médicale selon la revendication 2, dans laquelle chaque corps comprend une forme tétraédrique, chaque première extrémité comprend une extrémité faisant face vers l'extérieur et chaque seconde extrémité comprend une extrémité faisant face vers l'intérieur.

12. Sonde médicale selon la revendication 11, dans laquelle l'extrémité faisant face vers l'intérieur n'est pas alignée avec l'extrémité faisant face vers l'extérieur de telle sorte qu'une première ligne s'étendant perpendiculairement à la surface conductrice à partir d'un premier point au niveau d'un centre de la première extrémité est espacée d'une certaine distance par rapport à une seconde ligne parallèle à la première ligne et s'étendant à travers un second point au niveau d'un centre de la seconde extrémité.

13. Sonde médicale selon la revendication 11 ou la revendication 12, dans laquelle la pluralité d'électrodes sont fixées à chacun des éléments d'armature de telle sorte que chaque élément d'armature adjacent comprend une électrode, et dans laquelle la pluralité d'électrodes sont alignées dans une direction perpendiculaire à l'axe longitudinal.

14. Sonde médicale selon la revendication 11, le corps de chaque électrode de la pluralité d'électrodes étant fixé à l'élément d'armature respectif de telle sorte qu'un premier bord de la surface conductrice est orienté perpendiculaire à l'élément d'armature.

15. Sonde médicale selon la revendication 13, dans laquelle une première électrode de la pluralité d'électrodes est fixée à l'élément d'armature respectif dans une première orientation de telle sorte qu'un premier bord de la surface conductrice est au niveau d'une extrémité distale du corps, et
dans laquelle une seconde électrode de la pluralité d'électrodes est fixée à un élément d'armature adjacent de la pluralité d'éléments d'armature dans une seconde orientation de telle sorte qu'un premier bord de la surface conductrice est au niveau d'une extrémité proximale du corps, facultativement
dans laquelle la pluralité d'électrodes sont fixées à la pluralité d'éléments d'armature de telle sorte que la pluralité d'électrodes sont fixées à des éléments d'armature adjacents dans première et seconde orientations alternées.
